# EUROPÄISCHE PATENTSCHRIFT

(11) **EP 1 861 036 B1**
(45) Veröffentlichungstag und Bekanntmachung des Hinweises auf die Patenterteilung: **08.06.2016**
(21) Anmeldenummer: 06722613.4
(22) Anmeldetag: 16.03.2006
(51) Int. Cl.: A61B 90/00

(54) **PATIENTENAUFLAGE**
PATIENT SUPPORT
SUPPORT POUR PATIENT

(30) Priorität: 22.03.2005 DE 102005013151
(43) Veröffentlichungstag der Anmeldung: 05.12.2007
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2006/000456
(87) Internationale Veröffentlichungsnummer: WO 2006/099841

(56) Entgegenhaltungen:
- US-A- 5 297 303
- US-A- 5 569 266
- US-A- 5 865 181
- US-A1- 2001 039 378
- US-A1- 2002 156 365
- US-B1- 6 446 286

## Beschreibung

Die Erfindung betrifft eine Patientenauflage gemäß dem Oberbegriff des Patentanspruchs 1.

Patientenlagerungen mit einer Brustfixierungseinrichtung, einer Biopsie-Einrichtung oder Markier-Einrichtung sind bekannt. Hierbei weist die Patientenlagerung in einer oberen Auflagerplatte zwei Öffnungen auf, in welche die zu untersuchenden Brüste der Patientin frei hängend eingebracht werden können, Die obere Auflagerplatte stützt sich im Abstand von mehreren Zentimeter mit Hilfe einer Mehrzahl (z. B. vier) von Stützsäulen auf einer unteren Basisplatte ab.

Die DE 196 26 286 C2 zeigt eine MRI-Brustspule mit einer Einrichtung zum Fixieren einer zu biopsierenden Brust. Diese Einrichtung weist eine Patientenlagerung mit einer oberen Auflagerplatte mit zwei Öffnungen und zu einander beabstandeten Andrückelementen für eine Brust auf. In den Zwischenraum zwischen diesen Andrückelementen wird eine Brust eingebracht und durch ein Zusammenfahren der Andrückelemente wird die Brust schließlich fixiert und kann z. B. biopsiert werden.

Aus der US 2001/0039378 A1 ist eine MRI-Aufnahmevorrichtung für die weibliche Brust bekannt, bei der plattenförmige Fixiereinrichtungen entweder in Längsrichtung des Patienten (Kopf/Fuß) oder senkrecht hierzu angeordnet sind und bewegt werden. Im Einzelfall ist ein optimaler, d. h. möglichst kurzer Zugang zu den medizinisch vornehmlich interessierenden Gewebebereiche der Brust bei dieser Art der Fixierung nicht durchführbar.

Der Erfindung liegt die Aufgabe zugrunde eine Patientenauflage mit einer oberen Auflagerplatte und einer zu ihr horizontal beabstandeten Basisplatte zu schaffen, bei welcher der Zugangsbereich, d. h. die Zugänglichkeit zu der/die in den Raum zwischen Auflagerplatte und Basisplatte hängenden Brust/Brüste für diagnostische und/oder interventionale Maßnahmen, bei der Brust fixierende Einrichtungen verwendet werden können, bedeutend vergrößert wird.

Diese Aufgabe wird durch die Merkmale des Patentanspruches 1 gelöst.

Die Vorteile der Erfindung bestehen insbesondere darin, dass bei der erfindungsgemäßen Patientenlagerung die Möglichkeit zur Aufnahme einer Einrichtung zum Fixieren einer Brust, - mit oder ohne Einrichtung zum Biopsieren / Markieren einer Störung im Gewebegefüge innerhalb der Brust -, geschaffen wird, die für die genannte Einrichtung einen sehr großen Schwenkwinkelbereich um eine vertikale (senkrechte) Schwenkachse ermöglicht. Im vorderen Teil des Zwischenraumes zwischen oben liegender Auflagerplatte und unten liegender Basisplatte ist, - in Längsachseneinrichtung der Auflagerplatte gesehen - vom Bereich öffnung oder Aussparung für die Brust bis zum Anfang (Kopfauflagebereich) der Kopfauflagerfläche der

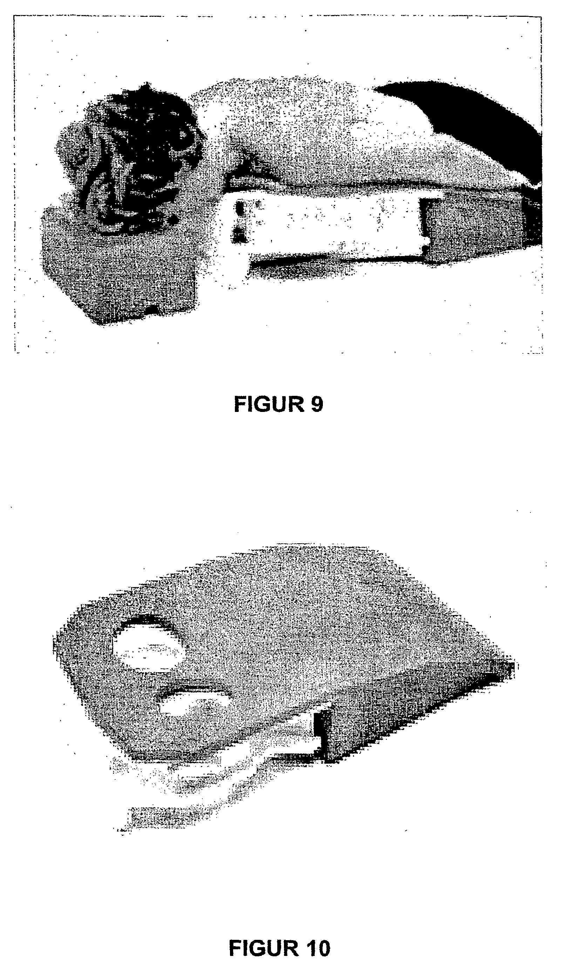

Auflagerplatte hin, ein oder mehrere, den Zwischenraum in horizontaler und vertikaler Richtung durchquerende(r), eine Abstützfunktion für die Auflagerplatte ausübende(r), schmale(r) und kurze(r) Stützkörper vorhanden. In vorteilhafter Weise kann die obere Auflagerplatte trotzdem in ihrer Länge so lang ausgeführt werden, dass auch noch der Kopf, oder zumindest mit ihrem Oberkörper auf der Patientenauflage aufliegenden Patientin, sich direkt oder indirekt abstützen (aufliegen) kann. Hierdurch wird in vorteilhafter Weise keine weitere Kopfstütze außerhalb (vor) der Patientenlagerung benötigt, welche den Zugang zu der fixierten Brust in der Richtung Kopf-Fuß unmöglich macht (siehe auch Stand der Technik dargestellt in den Figuren 10 und 11). In weiterer vorteilhafter Weise kann die Patientenauflage z.B. bei der Computertomographie (CT), Kemspintomographie (MRI) oder Positronen-Emissions-Tomographie (PET) eingesetzt werden. Die erfindungsgemäße Patientenauflage erlaubt in erfinderischer Weise die Anordnung einer oder mehrerer Empfangs- Flächenspulen, ohne dass der Rotationsbereich einer zu verwendenden Fixier- und/oder Biopsiereinrichtung wesentlich verkleinert werden muss.

Die Empfangs- Flächenspulen können in oder an der Basisplatte horizontal ausgerichtet befestigt werden. Auch können horizontal ausgerichtete Empfangs- Flächenspulen direkt oder indirekt - z.B. auch horizontal und/oder vertikal verschiebbar - an der Auflagerplatte befestigt sein.

Eine "Einrichtung zum Fixieren einer Brust, mit- oder ohne Halterung für ein medizinisches Werkzeug (Biopsier-, Markiereineinrichtung)" kann auf einfache Weise in die Patientenauflage eingesetzt und aus ihr entfernt werden.

Ein Ausführungsbeispiel der Erfindung wird in der Zeichnung dargestellt und wird im Folgenden näher beschrieben.

Es zeigen
Figur 1, eine Draufsicht auf die erfindungsgemäße Patientenauflage mit einer Ansicht auf eine Aufnahmevertiefung -(z.B. rechteckige)- in der Basisplatte für eine Befestigungsplatte für eine "Einrichtung zum Fixieren einer Brust, mit- oder ohne Halterung für ein medizinisches Werkzeug (Biopsier--, Markiereineinrichtung)" für eine Brust und/oder eine oder mehrere horizontal ausgerichtete MRI- Empfangs-Flächenspulen ,
Figur 2, eine Draufsicht nach Figur 1, jedoch mit der in die Aufnahmevertiefung eingelegten Befestigungsplatte,

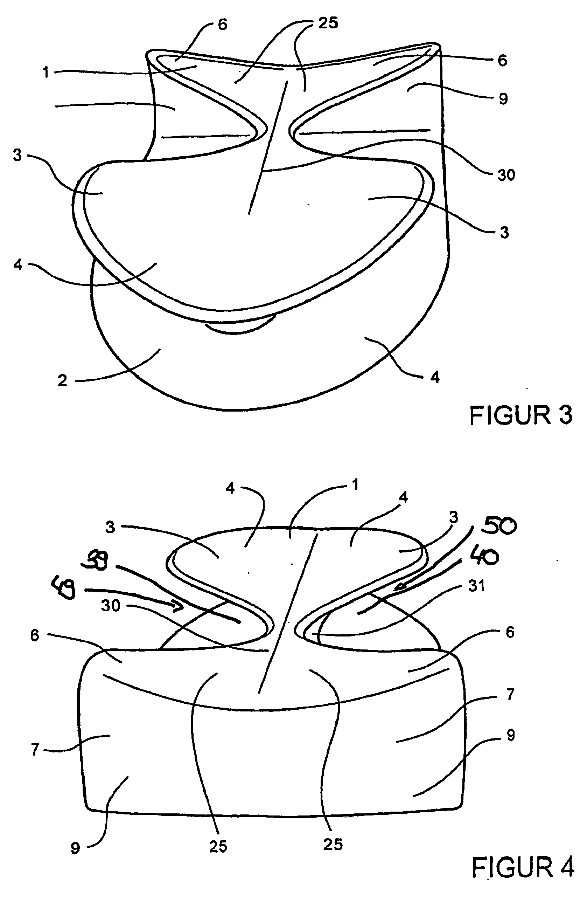

Figur 3, eine Vorderansicht der Patientenauflage nach Figur 1 mit Ansicht auf die erfindungsgemäße vordere Auflagerfläche mit einem fliegend gelagerten Kopfstück mit einer Kopfauflagefläche, die in Patientenfußrichtung in einen schmalen Steg übergeht und schließlich am Ende der Auflagerplatte über sein Oberkörperauflagerstück mit dem sich in vertikaler und horizontaler Richtung erstreckenden Endplatte verbunden ist,
Figur 4, eine Stimansicht auf ein die Enden von Auflagerplatte und Basisplatte verbindendes, sich in vertikaler Richtung erstreckende Endplatte der Patientenauflage,
Figur 5, eine Draufsicht auf die Oberseite der Befestigungsplatte mit zwei konischen Aufnahmebohrungen mit je einer Innenverzahnung,
Figur 6, eine Ansicht auf die Unterseite der Befestigungsplatte entsprechend Figur 5, mit je einer jeweils zur Aufnahmebohrung konzentrisch in die Unterseite der Befestigungsplatte eingearbeitete ringförmige Nut zur Aufnahme je einer Empfangs-Oberflächenspule,
Figur 7, eine rechte Seitenansicht auf die Patientenauflage mit einer, mit der Vorderseite ihres Oberkörper auf der Auflagerplatte aufliegenden Patientin. Mit außerdem einer parallel zur Längsachse der Auflagerplatte verdrehfest ausgerichteten Einrichtung zum Fixieren einer Brust, mit zwei in einem einstellbaren Abstand zueinander bringbaren Fixiergittem und mit einer Einrichtung zum Biopsieren / Markieren einer Störung im Gewebegefüge innerhalb der Brust,
Figur 8, eine Ansicht zwischen der Unterseite des Kopfstückes der Kopfauflagerplatte und der oberen Fläche der Basisplatte auf eine Einrichtung zum Fixieren einer Brust mit einer Einrichtung zum Biopsieren / Markieren deren entlang ihrer Längsachse aufgespannten vertikalen Ebene die, eine entlang der Längs- und Symmetrieachse der Auflagerplatte aufgespannte vertikale Ebene, in einem wählbaren Schnittwinkel schneidet,
Figuren 9 und 10 zeigen Patientenauflagen des Standes der Technik.

Bei der Computertomographie (CT) handelt es sich um ein diagnostisches Verfahren, bei dem die zu untersuchende Region durch ein eingegrenztes Röntgenstrahlenbündel von einer sich um den Körper rotierenden Röntgenröhre durchstrahlt wird.

Bei der Kernspintomographie (MRI) werden Aufnahmen des menschlichen Körpers ohne Anwendung ionisierender Strahlen (also ohne Anwendung von Röntgenstrahlung oder Anwendung radioaktiver Stoffe) erzeugt.

Die Positronen-Emissions-Tomographie (PET) ist ein hochempfindliches Verfahren, mit dem Stoffwechselvorgänge im Körper sichtbar gemacht werden, können.

Durch die CT-, MRI-, PET- Untersuchung kann die Lage einer Anomalie in einer fixierten Brust in einem rechtwinkligen Koordinatensystem exakt ausgemessen werden. Diese Werte können außerhalb oder innerhalb der MRI-Spule auf ein medizinisches Werkzeug, -z. B. eine Biopsienadel-, an einer anhand von Skalen horizontal- und vertikal einstellbaren Werkzeughalterung manuell oder motorisch voreingestellt werden. Die voreingestellte medizinische Werkzeughalterung kann auf einfache Weise in die Patientenlagerung eingesetzt werden

Eine Patientenauflage 1 besteht aus einer sich in horizontaler Richtung erstreckender Basisplatte 2 und einer oberhalb von ihr, in einem Abstand h angeordnete, sich in horizontaler Richtung erstreckenden Auflagerplatte 3. Zwischen ihnen ist ein Zwischenraum 14 vorgesehen. Der Abstand h (z.B. 80-100mm) zwischen der Unterseite 27 der Auflagerplatte 3 und der Oberseite 29 der Basisplatte 2 muss nicht über die gesamte Länge der Patientenauflage 1 gleich sein. Eine Längs- und Symmetrieachse 20 teilt die äußere Oberfläche der Auflagerplatte 3 in eine linke und in eine rechte Teilfläche auf. Sämtliche Bestandteile der Patientenauflage 1 bestehen aus einem MR-tauglichen Material, z.B. Kunststoff.

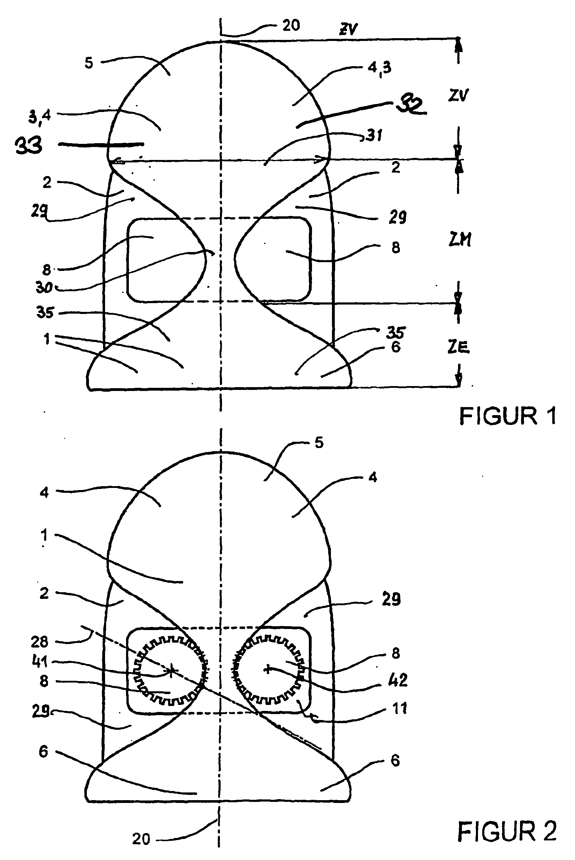

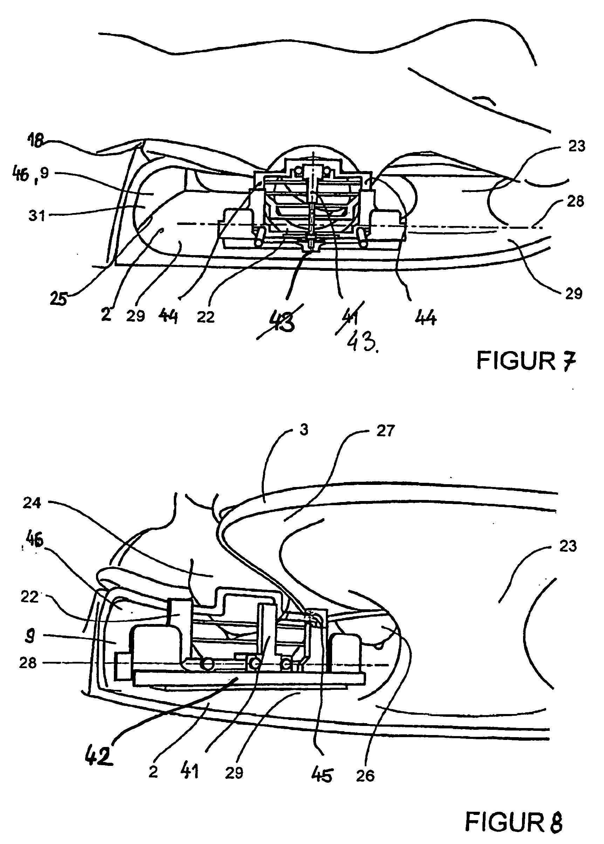

Die Auflagerplatte 3 weist im Wesentlichen entlang ihrer Längsachse 20, beginnend vom Kopfende, in etwa drei ausgeprägte Zonen auf:
1. eine vordere Zone ZV mit einer Kopfauflagerfläche 4 eines Kopfstückes 5. Sie erstreckt sich beispielsweise über ca. 35% der Länge der Auflagerplatte 3. Die vordere Zone ZV dient als Aufliegfläche für den Kopf und die Schulterpartie (siehe Figur 7) einer Patientin. Vorzugsweise ist/sind unter der vorderen Zone ZV der Auflagerplatte 3, also im vorderen Teil des Zwischenraumes 14, entlang oder in einem seitlichen Abstand von einer die Längs- und Symmetrieachse 20 senkrecht aufgespannten Vertikalebene ein oder mehrere schmale und ,-bezogen auf die Länge der Patientenauflage-, kurze Stützkörper (Stützsäule) 23 vorgesehen. Der/die Stützkörper 23 ist/sind mit der Unterseite der oberen Auflagerplatte 3 und der oberen Seite der Basisplatte 2 stoff- oder formschlüssig verbunden. Im Ausführungsbeispiel ist/sind oberer und/oder unterer Teil der Stützkörper 23 als Druckverteilerplatten ausgeführt, die mit der Unterseite der Auflagerplatte 3 bzw. mit der Oberseite der Basisplatte 2 stoff- oder formschlüssig verbunden sind. Die/der Stützkörper 23 erweitern aus diesem Grunde ihre oberen und unteren Enden pilzkopfförmig.
   Der/die Stützkörper 23 ist/sind in einem geringen Abstand, z.B. 70 bis 150 mm, vom Anfang der Kopfauflagerplatte 3 angeordnet Wird nur ein einziger Stützkörper 23 verwendet, so ist dieser an seiner schmalsten Stelle z.B. 10 bis 150 mm breit und z.B. 10 bis 200mm lang.
   Die Lage des/ der Stützkörpers 23 und die Höhe h des Zwischenraumes 14 sind so bestimmt, dass eine "Einrichtung zum Fixieren einer Brust, mit- oder ohne Halterung für ein medizinisches Werkzeug (Biopsier-, Markiereineinrichtung) 22", - im Folgenden kurz "Einrichtung... 22" genannt- ,jeweils in jeder ihrer Arbeitspositionen, - vorzugsweise um eine im jeweiligen Momentan-Zentrum der horizontal ausgerichteten Empfangs-Flächenspule errichtete Senkrechte-, um ca. 360° rotierbar ist Die Rotationsbewegung der Einrichtung... 22 wird also jeweils nicht durch Hindernisse behindert.
   Durch die vorteilhafte Plazierung des Stützkörpers 23 kann in vorteilhafter Weise die obere Auflagerplatte 3 der Patientenauflage 1 in cranialer Richtung so lang ausgeführt werden, dass auch noch der Kopf der mindestens mit ihrem Oberkörper auf der Auflagerplatte 3 liegenden Patientin, sich direkt oder indirekt auf der Patientenauflage 1 abstützen (aufliegen) kann. Hierdurch wird in vorteilhafter Weise keine außerhalb angeordnete, d.h. (vor) der Patientenlagerung angeordnete, Patientenauflage benötigt. Eine solche würde den Zugang zu einer, - in Kopf-Fuß-Richtung gesehen-, fixierten linken Brust 24 bzw. rechten Brust 26 in caudaler (Kopf-Fuß) Richtung unmöglich machen (siehe auch Stand der Technik, dargestellt in den Figuren 10 und 11).
2. Eine mittlere Zone ZM, die Brustkorb-Zone", schließt sich in caudaler Richtung der Auflagerplatte 3 der vorderen Zone ZV der Patientenauflage 1 an.
   Sie erstreckt sich beispielsweise über ca. 50% der Länge derAuflagerplatte 3. Sie hat beispielsweise die Form eines sich bis zu seiner halben Länge verjüngenden und anschließend wieder erweiternden Steges 30. Dieser dient als Auflagerfläche für das Brustbein der Patientin. Links und rechts der Längs- und Symmetrieachse 20 des Steges 30 erweitert sich der Steg 30 zu einem linken Ausschnitt 33 und ihm gegenüberliegend, rechts der Längs- und Symmetrieachse 20 zu einem rechten Ausschnitt 32. Der linke Ausschnitt 33 ist links außen offenen, der rechte Ausschnitt 32 ist rechts außen offen. Der Steg 30 kann z.B. an seiner schmalsten Stelle zwischen 40 mm und 100 mm breit sein. Die Umrisse der Ausschnitte 32 und 33 können die Form in einer horizontalen Ebene oder einer zu dieser nach oben oder nach unten hin geneigten Ebene, liegenden Parabel haben. Ihre Scheitelpunkte können gleichen oder unterschiedlichen Abstand von der Längs- und Symmetrieachse 20 aufweisen, vorzugsweise sind sie jedoch gleich. Diese sich nach außen hin weit öffnenden Ausschnitte 32 und 33 ermöglichen einerseits ein ganz tiefes und freies Durchhängen auch größerer Brüste vor ihrer Fixierung. Zusätzlich wird auch ein größerer hindemisfreier Blick auf die Einrichtung... 22 mit der fixierten Brust 24 bzw. 26 ermöglicht.
   Dieses ist deshalb so wichtig, weil die "Einrichtung zum Fixieren einer Brust mit der Einrichtung... 22 ohne die fixierte Brust um ihre senkrecht stehende Achse bis zu 360° stufenlos oder in kleinen Schritten rotiert (verdreht) werden und anschließend lösbar verdrehfest gemacht werden kann. Und
3. schließlich die Endzone ZE (Endfläche) der Auflagerplatte 3. Sie erstreckt sich, beginnend vom Ende der Auflagerplatte 3, über ca. 15 % der Länge der Auflagerplatte 3. Sie dient als Auflagefläche 6 für den, - in Fußrichtung gesehen -, sich an den Brustkorb anschließenden oberen Bauchabschnitt (siehe Figuren 1 und 7).
   Die Auflagefläche 6 der Auflagerplatte 3 endet an der sich in vertikaler und horizontaler Richtung erstreckenden Endplatte 9. Das Ende der Auflagerplatte 3 ist vorzugsweise mit dem oberen Teil 7 der Endplatte 9 stoff- oder formschlüssig verbunden. Der untere Teil 10 der Endplatte 9 ist mit dem Ende der Basisplatte 2 stoff- oder formschlüssig verbunden.

Durch die kotierte Projektion des linken Ausschnittes (33) bzw. des rechten Ausschnittes (32) in den Zwischenraum (14) wird auf die Oberseite (29) der Basisplatte (2) ein linker Flächenabschnitt (39) bzw. ein rechter Flächenabschnitt (40) gedanklich abgebildet. Im derart umgrenzten linken- (49) bzw. rechten Arbeitsraum (50) ist die Einrichtung zum Fixieren einer Brust, - mit oder ohne Halterung für ein medizinisches Werkzeug (Biopsier-, Markiereineinrichtung)-, 22 angeordnet.
Der Bereich unterhalb der Ausschnitte 32, 33 (linker- (49) bzw. rechten Arbeitsraum (50)) und dem Steg 30 ist also der Bereich in dem die Einrichtung... 22 angeordnet ist. Sie kann, wie schon oben ausgeführt, um eine senkrechte Achse um ca. 360° gedreht werden.
Die Drehbewegung (Rotationsbewegung) der Einrichtung... 22 wird in vorteilhafter Weise nicht durch Hindernisse behindert oder eingeschränkt.

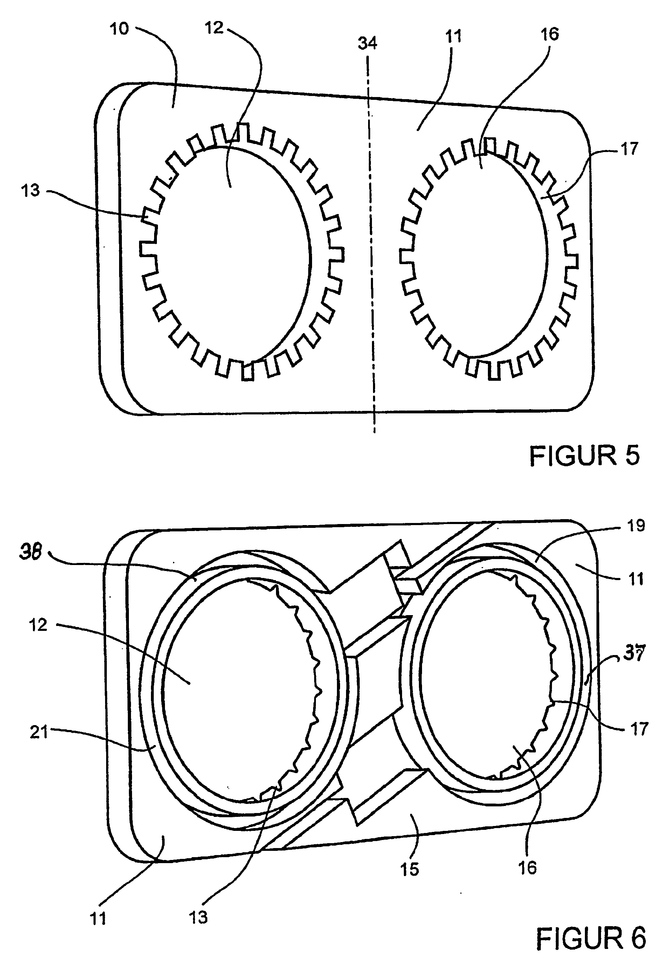

Eine quaderförmige Befestigungsplatte 11 mit z.B. einer Länge, die fast der gesamten Breite der Basisplatte 2 im Bereich des Steges 30 der Auflagerplatte 3 entspricht, ist herausnehmbar und formschlüssig in einer Aufnahmevertiefung 8 oder einem Aufnahmeloch 8 z.B. mittels Führung , -z.B. Flach- oder Schwalbenschwanführung-, befestigt. Aufnahmevertiefung 8 oder Aufnahmeloch 8 ist in der Basisplatte 2, von ihrer der oberen Seite ausgehend, eingearbeitet Die Befestigungsplatte 11 weist z.B. zwei kreisrunde durchgehende Aufnahmebohrungen 12; 16 vorzugsweise gleichen Durchmessers, auf. Die Zentren Aufnahmebohrungen 12; 16 und damit die Zentren der Empfangs-Flächenspulen 37,38, haben ungleiche Abstände a und b, - z. B. 3 bis 4 cm Differenz-, von der Quer-Mittellinie 34 der Befestigungsplatte 11. Diese Differenz zwischen den Abständen a und b ist begründet durch die vorteilhafte Anpassungsmöglichkeit auf unterschiedliche Breiten der Oberkörper und/oder des Brustabstandes der zu untersuchenden Patientinnen.
Die Aufnahmebohrungen 12; 16 sind z.B. je mit je einer Innenverzahnung 13; 17 versehen. Die Unterseite 15 der Befestigungsplatte 11 entsprechend Figur 5, ist mit je einer, jeweils zu ihrer Aufnahmebohrung 12; 17 konzentrisch in die Unterseite 15 der Befestigungsplatte 11 eingearbeitete ringförmige linke Nut 21 zur Aufnahme einer linken Empfangs-Flächenspule 38 und eine eingearbeitete ringförmige rechte Nut 19 zur Aufnahme einer rechten Empfangs-Flächenspule 37 versehen.

Damit die Einrichtung... 22 ohne die fixierte Brust um ihre senkrecht stehende Achse bis zu ca. 360° stufenlos oder in kleinen Schritten rotiert (verdreht) werden und anschließend lösbar verdrehfest gemacht werden kann, ist z.B. an der Unterseite der Basis-Platte 2 der Einrichtung... 22 form- oder stoffschlüssig ein Bund, z.B. in Form eines Kreisringes mit z.B. einem Außenkonus angebracht. Dieser hat z.B. einen außenkonischen Zahnkranz.
Der Bund kann mit einer an ihn angepassten Aufnahme in Formschluss gebracht werden. Bund und Aufnahme bilden also eine Kupplung. Diese Kupplung kann z.B. als schaltbare (wahlfrei lösbare) formschlüssige Kupplung oder kraftschlüssige Kupplung ausgeführt sein.
Im Ausführungsbeispiel wird eine schaltbare formschlüssige Kupplung, Zahnkupplung, verwendet.
Die Zähne des außenkonischen Zahnkranzes (Bund) können jeweils mit je einer Innenverzahnung 13; 17 der z.B. zwei konischen Aufnahmebohrungen 12; 16 (Aufnahmen) einer Befestigungsplatte 11 für die Einrichtung... 22 in und außer Eingriff gebracht, d.h. ein-und ausgekuppelt werden. Sie bilden also eine schaltbare, d.h. wahlfrei trennbare, formschlüssige Kupplung.
Zum Ein- und Auskuppeln und Drehen der Einrichtung... 22 um ihre vertikale Achse wird jene angehoben und somit ihre konische Außenverzahnung aus der zugeordneten Innenverzahnung 13;17 der ersten Aufnahmebohrung 12 bzw. der zweiten Aufnahmebohrung 16 zuerst außer Eingriff gebracht und anschließend um ihre senkrechte Achse um einem freiwählbaren Winkel verdreht (rotiert) und anschließend wieder in Eingriff gebracht wird. Wie schon ausgeführt, ist die anwendbare Kupplungsart ist nicht auf das Zahnkupplungsprinzip beschränkt. So könnte auch andere formschlüssige Kupplungsarten aber auch kraftschlüssige Kupplungen, z.B. Kegelreibkupplungen verwendet werden.

Die Befestigungsplatte 11 mit den zwei Aufnahmebohrungen 12, 16 kann auch kürzer ausgeführt sein, so dass sie quer zur Längsachse 20, geführt mittels einer Führung, verschoben und in einer gewünschten Stellung mittels einer reib- oder formschlüssig arbeitenden Arretiereinrichtung arretiert werden kann. Verschoben kann die Befestigungsplatte 11, um an Bauhöhe zu sparen, in der Aufnahmevertiefung 8 oder dem Aufnahmeloch 8.
Die Befestigungsplatte 11 kann auch mit nur mit einer Aufnahmebohrung 12 für nur eine MRI-Empfangs-Flächenspule 38. Es wäre also nur eine MRI-Empfangs-Flächenspule 38 notwendig, die sowohl für die Untersuchung an der linken Brust, wie auch an der rechten Brust verwendet wird, notwendig. Die entsprechend kurze Befestigungsplatte 11 müsste nur von der linken Seite auf die rechte Seite mittels einer Führung quer zur Längsachsenrichtung 20 verschoben werden.

Zusätzlich zu der sich horizontal erstreckenden unteren linken Empfangs-Flächenspule 38 und/oder der sich horizontal erstreckenden unteren rechten Empfangs-Flächenspule 38 in der Basisplatte 2 bzw. in der Befestigungsplatte 11 können noch eine weitere sich horizontal erstreckende obere linke- bzw. obere rechte Empfangs-Flächenspule angeordnet werden um die Bildqualität zu erhöhen. Diese eben genannten oberen Empfangs-Flächenspulen werden an der Auflagerplatte 2 direkt oder indirekt, z.B. an einem Polster auf der Patientenauflage 1 angebracht Die linke obere - und die linke untere Empfangs-Flächenspule bilden ein linkes horizontal ausgerichtetes Brustspulenpaar bzw. die obere rechte - und die untere rechte Empfangs-Flächenspule bilden ein rechtes horizontal ausgerichtetes Brustspulenpaar. Die Zentren des linken- bzw. des rechten Brustspulenpaares sollten möglichst auf einer gemeinsamen linken Senkrechten bzw. auf einer gemeinsamen rechten Senkrechten liegen. Die oberen Empfangs-Flächenspulen bestehen aus Metall und sind gegen elektrische Spannung und Wärme isoliert. Sie haben z.B. die Form eines Kreisringes, der an einer Stelle über seine gesamte Kreisringbreite durchtrennt ist, wodurch der Kreisring zwei Anschlussenden erhält. Die oberen Empfangs-Flächenspulen sind biegesteif ausgeführt. Sie sind vorzugsweise im Bereich ihrer Anschlussenden am Steg 30 befestigt elektrisch angeschlossen. Die oberen Empfangs-Flächenspulen ragen jeweils vom Steg 30 ausgehend links bzw. rechts in den linken Ausschnitt 32 bzw. in den rechten Ausschnitt 33, horizontal ausgerichtet, hinein.

Die Einrichtung zum Fixieren einer Brust, mit - oder ohne Halterung für ein medizinisches Werkzeug (Biopsier-, Markiereineinrichtung) 22 kann zwei zueinander beabstandete, vertikal ausgerichtete Empfangs-Flächenspulen (linke- und rechte Empfangs-Flächenspule) aufweisen. Die vertikalen Empfangs-Flächenspulen sind vorzugsweise an den beiden Fixiergittern angebracht.
In diesem Falle bilden obere horizontale Empfangs-Flächenspule, rechte vertikale Empfangs-Flächenspule, untere horizontale Empfangs-Flächenspule und linke vertikale Empfangs-Flächenspule miteinander ein Spulenarray.

Nachfolgend soll der Gegenstand der Anmeldung noch einmal zusammengefasst werden:
Die Patientenauflage1 besteht aus einer oberen Auflagerplatte 3 und einer unterhalb und beabstandet zu ihr angeordneten Basisplatte 2. Beide sind durch einen Zwischenraum 14 voneinander getrennt. Die Auflagerplatte 3 weist in ihren vorderen Teil ZV einen fliegend gelagerten Teil mit einer Kopfauflagefläche 4 auf. Im Bereich der Kopfauflagerfläche 4 der Auflagerplatte 3 ist zwischen deren Unterseite 27 und der Oberseite 29 der Basisplatte 2 die einzige, tragende, schmale Stützsäule 23 vorhanden. Sie ist mit der Oberseite 29 und der Unterseite 27 form- oder stoffschlüssig verbunden,

Die Stützsäule 23, ist, quer zur Längsachsenrichtung 20 der Auflagerplatte 2 gesehen im Vergleich zur Breite der Auflagerplatte 3 schmal (z.B. 1cm bis15cm breit) und -in Längsachsenrichtung 20 gesehen- bezogen auf die Länge derAuflagerplatte 3, kurz (z.B. 1cm bis 20cm lang).
Das Ende 25 der Basisplatte 2 ist mit dem Ende 18 der Auflagerplatte 3 mittels einer sich in horizontaler Richtung erstreckenden Endplatte 9 form- oder stoffschlüssig verbunden.
Die Richtungen der Längsachsen der Stützsäule 23 der Auflagerplatte 3 können in einem beliebigen Öffnungswinkel zu einander laufen, vorzugsweise ist er gleich Null Grad oder wenige Grade, z.B. bis plus/minus 10°.
Besonders ist es, wenn die vertikale Ebene entlang der Längsachse der Stützsäule 23 und die vertikale Ebene entlang der Längsachse 20 der Auflagerplatte 3 zusammenfallen.
Die vertikale Ebene entlang der Längsachse der Stützsäule 23 und die vertikale Ebene entlang der Längsachse 20 der Auflagerplatte 3 können aber auch einen horizontalen Abstand von einander haben.
Die obere Auflagerplattenfläche 6 der Auflagerplatte 3 weist beginnend von ihrem Anfang bis zu ihrem Ende 18 mindestens drei aufeinander folgende Flächenzonen auf: eine vordere Zone ZV, eine auf sie folgende mittlere Zone ZM eine auf diese folgende Endzone EZ.
In Richtung Ende 18 (=Patientenfußrichtung) der Auflagerplatte 3 gesehen, verringert sich die Breite 31 der Auflagerplattenfläche 6 der Auflagerplatte 3 von ihrer breitesten Stelle aus in der vorderen Zone ZV fortlaufend bis zu einem schmalen Zwischenstück 30 in der mittleren Zone ZM und von da aus verbreitert sie sich fortlaufend bis zum Ende der Auflagerplatte 3 in der Endzone EZ wieder. So wird der linke Ausschnitt 33 und der rechte Ausschnitt 32 gebildet. Der linke Ausschnitt 33 und der rechte Ausschnitt 32 erstrecken sich in horizontaler Richtung, - vorzugsweise sind sie horizontal-, und sind symmetrisch zu der Längs- und Symmetrieachse 20 angeordnet
Die Reduzierung der Breite 31 der Auflagerplattenfläche 6 kann nach den Gesetzmäßigkeiten einer ebenen Kurve erfolgen.

Die ebene Kurve kann eine Gerade oder eine Parabel oder ein Kreis oder eine Ellipse oder ein Hyperbel sein.
Die ebene Kurve kann wahlweise aus Teilen einer Gerade und/oder einer Parabel und/oder eines Kreises und/oder einer Ellipse und/oder einer Hyperbel zusammengesetzt sein.

Im Zwischenraum 14 wird durch kotierte Projektion des linken Ausschnittes 33 bzw. des rechten Ausschnittes 32 auf die Oberseite 29 der Basisplatte 2 abgebildeten linken Flächenabschnittes 39 bzw. rechten Flächenabschnitt 40 ein linker Arbeitsraum 49 bzw. ein rechter Arbeitsraum 50 gebildet. Im Arbeitsräum49 bzw.50 ist die Einrichtung zum Fixieren einer Brust 22 angeordnet. Die Einrichtung zum Fixieren einer Brust 22 stützt sich direkt oder indirekt auf der Basisplatte 2 ab und ist mit ihr direkt oder indirekt formschlüssig oder stoffschlüssig verbunden.
Im linken Arbeitsraum 49 bzw. im rechten Arbeitsraum 50 kann zusätzlich zur Einrichtung zum Fixieren einer Brust 22 die Halteeinrichtung für ein medizinisches Werkzeug angeordnet sein, wobei sich die Halteeinrichtung 41 für das medizinische Werkzeug auf der Basisplatte 2 direkt oder indirekt abstützt. Die Halteeinrichtung 41 kann mit der Einrichtung zum Fixieren einer Brust 22 formschlüssig oder stoffschlüssig verbunden sein.
Die Einrichtung zum Fixieren einer Brust 22 kann innerhalb des linken Arbeitsraumes 49 bzw. innerhalb des rechten Arbeitsraumes 50 um eine senkrechte Achse 42,43 bzw. des rechten drehbar und arretierbar angeordnet sein.
Im linken Arbeitsraum 49 bzw. im rechten Arbeitsraum 50 können mindestens eine linke und/oder eine rechte, horizontal ausgerichtete MRI-taugliche Empfangs-Flächenspule (37,38) angeordnet sein.
Im linken Arbeitsraum 49 bzw. im rechten Arbeitsraum 50 kann innerhalb oder auf oder unter der der Basisplatte 2 eine Befestigungseinrichtung 11 zur Aufnahme für mindestes eine MRI-taugliche Empfangs-Flächenspule 37,38 angeordnet sein.
Die Befestigungseinrichtung 11 kann quer zur Längs- und Symmetrieachse 20 horizontal verschiebbar sein.
Die Befestigungseinrichtung 11 kann eine oder zwei Einrichtungen 12,13 zur Aufnahme einer Einrichtung zum Fixieren einer Brust 22 mit oder ohne Halteeinrichtung 41 für ein medizinisches Werkzeug aufweisen.
Die Einrichtung zum Fixieren einer Brust 22,- mit oder ohne Halteeinrichtung 41-, kann mittels einer wahlfrei lös- und schließbaren, kraft- oder formschlüssigen Kupplung 12,13 direkt- oder indirekt 11 mit der Basisplatte 2 verbunden sein.

Im linken Arbeitsraum 49 bzw. im rechten Arbeitsraum 50 können zwei zueinander parallele, vertikal ausgerichtete, voneinander beabstandete MRI-taugliche Empfangs-Flächenspulen 41,45 angeordnet sein. Eine oder beide vertikal ausgerichtete/n, voneinander beabstandete/n MRI-taugliche Empfangs-Flächenspule/n 41,45 können an der Einrichtung zum Fixieren einer Brust 22 befestigt sind.
Eine oder beide vertikal ausgerichtete/n, voneinander beabstandete/n MRI-taugliche/n Empfangs-Flächenspule/n 41,45 könnten aber auch an der Innenseite (46) der Endplatte (9) befestigt sein.
Die Patientenauflage kann eine obere linke und/oder rechte MRI-taugliche Empfangs-Flächenspule aufweisen, die in den linken Arbeitsraum 49 und/oder in den rechten Arbeitsraum 50 hineinwirken. Die obere linke und/oder rechte MRI-taugliche Empfangs-Flächenspule direkt- oder indirekt an der Auflagerplatte (3) befestigt sein.

**Bezugszeichenliste**

| | | | |
|---|---|---|---|
| 1 | Patientenauflage | 35 | Endfläche (6) |
| 2 | Basisplatte | 36 | - |
| 3 | Auflagerplatte | 37 | Empfangs-Flächenspule, untere, rechte, horizontale |
| 4 | Kopfauflagerfläche | | |
| 5 | Kopfstück | 38 | Empfangs-Flächenspule, untere, linke, horizontale |
| 6 | Auflagefläche | | |
| 7 | Teil, oberer | 39 | Projektions-Flächenbereich, linker |
| 8 | Aufnahmevertiefung, Aufnahmeloch | 40 | Projektions-Flächenbereich, rechter |
| 9 | Endplatte | 41 | Halteeinrichtung für ein medizinisches. Werkzeug |
| 10 | Teil, unterer | | |
| 11 | Befestigungsplatte | 42 | Drehachse, linke, senkrechte |
| 12 | Aufnahmebohrung | 43 | Drehachse, rechte, senkrechte |
| 13 | Innenverzahnung | 44 | Empfangs-Flächenspule, rechte, vertikale |
| 14 | Zwischenraum | | |
| 15 | Unterseite | 45 | Empfangs-Flächenspule, linke, vertikale |
| 16 | Aufnahmebohrung, konisch | | |
| 17 | Innenverzahnung | 46 | Endstück-Innenfläche |
| 18 | Ende (3) | | |
| 19 | Nut, rechte | | |
| 20 | Längs- und Symmetrieachse | | |
| 21 | Nut, rechte | | |
| 22 | Einrichtung zum Fixieren einer Brust | 49 | Arbeitsraum, linker |
| 23 | Stützkörper (Stützsäule) | 50 | Arbeitsraum, rechter |
| 24 | Brust, linke, fixierte | | |
| 25 | Ende (2) | | |
| 26 | Brust, rechte, fixierte | | |
| 27 | Unterseite (2) | | |
| 28 | Längsachse (22) | ZV | = vordere Zone |
| 29 | Oberseite (2) | ZM | = mittlere Zone |
| 30 | Steg | ZE | = Endzone |
| 31 | Breite, veränderlich, obere (4) | | |
| 32 | Ausschnitt, rechter | h | = Abstand |
| 33 | Ausschnitt, linker | | |
| 34 | Quer-Mittellinie | | |

## Patentansprüche

1. Patientenauflage mit einer oberen Auflagerplatte (3) mit einer Auflagerplattenfläche (31) und einer unterhalb und beabstandet zu ihr angeordneten Basisplatte (2), wobei sich die Auflagerplatte (3) auf der Basisplatte (2) abstützt, wobei zwischen der Unterseite der Auflagerplatte (3) und der Oberseite der Basisplatte (2) ein Zwischenraum (14) gebildet wird, wobei die obere Auflagerplattenfläche (31) der Auflagerplatte (3) von ihrem Anfang bis zu ihrem Ende (18) mindestens drei aufeinander folgende Flächenzonen aufweist: eine vordere Zone (ZV), eine auf sie folgende mittlere Zone (ZM) und eine auf diese folgende Endzone (EZ) und die mittlere Zone (ZM), die sich zu einem schmalen Zwischenstück (30) verjüngt und anschließend wieder verbreitert und so einen linken Ausschnitt (33) und einen rechten Ausschnitt (32) bildet, die jeweils horizontal ausgerichtet und symmetrisch zu der Längs- und Symmetrieachse (20) der Auflagerplatte (3) angeordnet sind, schließlich eine Einrichtung zum Fixieren einer Brust (22) angeordnet ist und im Zwischenraum (14) im Bereich, eines durch kotierte Projektion des linken Ausschnittes (33) bzw. des rechten Ausschnittes (32) auf die Oberseite (29) der Basisplatte (2) abgebildeten linken Flächenabschnittes (39) bzw. rechten Flächenabschnitt (40) die Einrichtung zum Fixieren einer Brust (22) angeordnet ist, dass die Einrichtung zum Fixieren einer Brust (22) sich jeweils auf der Basisplatte (2) direkt oder indirekt abstützt, dass die Einrichtung zum Fixieren einer Brust (22) direkt oder indirekt formschlüssig oder stoffschlüssig mit der Basisplatte (2) verbunden ist und eine Stütze (23) vorhanden ist,
**dadurch gekennzeichnet, dass** in der vorderen Zone (ZV) im Zwischenraum (14) sich die einzige, tragende Stütze (23) befindet, die quer zur Längsachsenrichtung gesehen, im Vergleich zur Breite der Auflagerplatte (3) schmal ist und in Längsachsenrichtung gesehen im Vergleich zur Länge der Auflagerplatte (3) kurz ist und dass jeweils die Einrichtung zum Fixieren einer Brust (22) um eine senkrechte Achse (42, 43) innerhalb des abgebildeten linken Flächenabschnittes (39) bzw. rechten Flächenabschnitt (40) des linken Ausschnittes (33) bzw. des rechten Ausschnittes (32) drehbar und arretierbar angeordnet ist.

2. Patientenauflage nach Anspruch 1, **dadurch gekennzeichnet, dass** eine in den, durch kotierte Projektion des linken Ausschnittes (33) bzw. des rechten Ausschnittes (32) auf die Oberseite (29) der Basis-platte (2) umgrenzten Raum (14) hineinwirkende, obere linke - (47) und/oder rechte MRI-taugliche Empfangs-Flächenspule (48) direkt-oder indirekt an der Auflagerplatte (3) befestigt sind.

3. Patientenauflage nach Anspruch 1 oder 2, **dadurch gekennzeich-net,** dass die Einrichtung zum Fixieren einer Brust (22) mit oder ohne einer Halteeinrichtung (41) für ein medizinisches Werkzeug mittels einer wahlfrei lös- und schließbaren, kraft- oder formschlüssigen Kupplung (12, 13) direkt- oder indirekt (11) mit der Basisplatte (2) verbunden ist.

4. Patientenauflage nach Ansprüchen 1 oder 2, **dadurch gekennzeichnet, dass** im durch kotierte Projektion des linken Ausschnittes (33) bzw. des rechten Ausschnittes (32) auf die Oberseite (29) der Basisplatte (2) abgebildeten linken Flächenabschnittes (39) bzw. den rechten Flächenabschnittes (40), innerhalb oder auf oder unter der Basisplatte (2) eine Befestigungseinrichtung (11) zur Aufnahme für mindestens eine horizontal ausgerichtete MRI-taugliche Empfangs-flächenspule (37, 38) angeordnet ist.

5. Patientenauflage nach Anspruch 4, **dadurch gekennzeichnet, dass** die Befestigungseinrichtung (11) horizontal und quer zur Längs- und Symmetrieachse (20) verschiebbar ist.

6. Patientenauflage nach Anspruch 4, **dadurch gekennzeichnet, dass** die Befestigungseinrichtung (11) eine Anzahl von Einrichtungen (12, 16) zur Aufnahme einer Einrichtung zum Fixieren einer Brust (22), mit oder ohne Halteeinrichtung (41), für ein medizinisches Werkzeug aufweist.

7. Patientenauflage nach Anspruch 4, **dadurch gekennzeichnet, dass** die Einrichtung zum Fixieren einer Brust (22), mit oder ohne einer Halteeinrichtung (41), für ein medizinisches Werkzeug mittels einer wahlfrei lös- und schließbaren, kraft- oder formschlüssigen Kupplung (12, 13) mit der Befestigungseinrichtung (11) verbunden ist.

8. Patientenauflage nach Ansprüche 1, 3 bis 5 oder 2 bis 5, **dadurch gekennzeichnet, dass** im Zwischenraum (14) im, eines durch kotierte Projektion des linken Ausschnittes (33) bzw. des rechten Ausschnittes (32) auf die Oberseite (29) der Basisplatte (2) abgebildeten linken Flächenabschnitt (39) bzw. den rechten Flächenabschnitt (40) eine Halteeinrichtung (41) für ein medizinische Werkzeug angeordnet ist, dass sich die Halteeinrichtung (41) für das medizinische Werkzeug auf der Basisplatte (2) direkt oder indirekt abstützt.

9. Patientenauflage nach Anspruch 8, **dadurch gekennzeichnet, dass** die Halteeinrichtung für ein medizinisches Werkzeug (41) mit der Einrichtung zum Fixieren einer Brust (22) formschlüssig oder stoffschlüssig verbunden ist.

10. Patientenauflage nach Ansprüchen 1, 3 bis 9 oder 2 bis 9, **dadurch gekennzeichnet, dass** im, durch kotierte Projektion des linken Ausschnittes (33) bzw. des rechten Ausschnittes (32) auf die Oberseite (29) der Basisplatte (2) abgebildeten linken Flächenabschnitt (39) bzw. den rechten Flächenabschnitt (40) innerhalb oder auf oder unter der Basisplatte (2) mindestens eine linke und/oder eine rechte, horizontal ausgerichtete MRI-taugliche Empfangs-Flächenspule-(37, 38) angeordnet sind.

11. Patientenauflage nach Anspruch 4, **dadurch gekennzeichnet, dass** an der Befestigungseinrichtung (11) mindestens eine horizontal ausgerichtete MRI-taugliche Empfangs-Flächenspule (37, 38) angeordnet ist.

12. Patientenauflage nach Anspruch11, **dadurch gekennzeichnet, dass** eine oder beide vertikal ausgerichtete/n, voneinander beabstandete/n MRI-taugliche Empfangs-Flächenspule/n (41, 45) an der Einrichtung zum Fixieren einer Brust (22) angeordnet und befestigt sind.

13. Patientenauflage nach Anspruch 1 oder 2, **dadurch gekennzeichnet, dass** im, durch kotierte Projektion des linken Ausschnittes (33) bzw. des rechten Ausschnittes (32) auf die Oberseite (29) der Basis-platte (2) umgrenzten Raum (14) zwei zueinander parallele, vertikal ausgerichtete, voneinander beabstandete MRI-taugliche Empfangs-Flächenspulen (41, 45) angeordnet sind.

14. Patientenauflage nach einem der vorhergehenden Ansprüche, **dadurch gekennzeichnet, dass** das Ende (25) der Basisplatte /2) mit dem Ende (18) der Auflagerplatte (3) mittels einer sich in horizontaler und vertikaler Richtung erstreckenden Endplatte (9) form-oder stoffschlüssig verbunden ist.

15. Patientenauflage nach Anspruch 14, **dadurch gekennzeichnet, dass** eine oder beide vertikal ausgerichtete/n, von einander beabstandete/n MRI-taugliche/n Empfangs-Flächenspule/n (41, 45) an einer Innenseite (46) der am Ende (18) der Auflagerplatte (3) befindlichen Endplatte (9) befestigt sind.

## Claims

1. Patient support comprising an upper support plate (3) with a support plate surface (31) and a base plate (2) that is mounted below and spaced therefrom, the support plate (3) being supported on the base plate (2) and an interspace (14) being formed between the underside of the support plate (3) and the upper side of the base plate (2) , the upper support plate surface (31) of the support plate (3) having at least three successive surface zones from its beginning to its end (18). a front zone (ZV), a subsequent middle zone (ZM), and a subsequent end zone (EZ), and the middle zone (ZM), which tapers to a narrow intermediate piece (30) and subsequently widens again and thus forms a left section (33) and a right section (32), which are in each case oriented horizontally and are symmetrical to the longitudinal and symmetry axis (20) of the support plate (3), finally a device for fixing a breast (22) is disposed and the device for fixing a breast (22) is disposed in the interspace (14), in the region of a left area-section (39) and right area-section (40) in each case, which are depicted by topographic projection of the left section (33) and/or of the right section (32) in each case on the top side (29) of the base plate (2), such that the device for fixing a breast (22) is directly or indirectly supported on the base plate (2), that the device for fixing a breast (22) is directly or indirectly connected in an interlocking or integral manner to the base plate (2) and a support (23) is present,
**characterised in that** in the front zone (ZV) in the interspace (14) the only supporting prop (23) is located, which, seen transversely to the longitudinal axial direction, is narrow in comparison to the width of the support plate (3) and, seen in the longitudinal axial direction, is short in comparison to the length of the support plate (3) and **in that** the device for fixing a breast (22) is in each case disposed so as to be rotatable and lockable about a vertical axis (42, 43) within the depicted left area-section (39) or right area-section (40) of the left section (33) or of the right section (32).

2. Patient support according to claim 1, **characterised in that** an upper left (47) and/or right MRI-compatible receiver coil (48), which acts into the space (14) delimited by topographic projection of the left section (33) or of the right section (32) onto the top side (29) of the base plate (2) is fastened directly or indirectly on the support plate (3).

3. Patient support according to claim 1 or 2, **characterised in that** the device for fixing a breast (22), with or without a retaining device (41) for a medical instrument, is directly or indirectly (11) connected to the base plate (2) by means of an optionally detachable and closable frictional or interlocking coupling (12, 13).

4. Patient support according to claim 1 or 2, **characterised in that,** in the left area-section (39) or right area-section (40) formed by topographic projection of the left section (33) or of the right section (32) onto the top side (29) of the base plate (2), a fastening device (11) for receiving at least one horizontally oriented MRI-compatible receiver coil (37, 38) is disposed within or on or below the base plate (2).

5. Patient support according to claim 4, **characterised in that** the fastening device (11) is displaceable horizontally and transversely to the longitudinal and symmetrical axis (20).

6. Patient support according to claim 4, **characterised in that** the fastening device (11) comprises a number of devices (12, 16) to receive a device for fixing a breast (22), with or without a retaining device (41) for a medical instrument.

7. Patient support according to claim 4, **characterised in that** the facility for fixing a breast (22), with or without a retaining device (41) for a medical instrument, is connected to the fastening device (11) by means of an optionally detachable and closable frictional or interlocking coupling (12, 13).

8. Patient support according to claims 1, 3 to 5 or 2 to 5, **characterised in that,** in the intermediate space (14) in the left area-section (39) or right area-section (40) depicted by topographic projection of the left section (33) or of the right section (32) onto the top side (29) of the base plate (2), a retaining device (41) for the medical instrument is disposed, and **in that** the retaining device (41) for the medical instrument is directly or indirectly supported on the base plate (2).

9. Patient support according to claim 8, **characterised in that** the retaining device for a medical instrument (41) is interlockingly or integrally connected to the device for fixing a breast (22).

10. Patient support according to claims 1, 3 to 9 or 2 to 9, **characterised in that** in the left area-section (39) or right area-section (40) depicted by topographic projection of the left section (33) or of the right section (32) onto the top side (29) of the base plate (2), at least one left and/or right, horizontally oriented MRI-compatible receiver coil (37, 38) is disposed within or on or below the base plate (2).

11. Patient support according to claim 4, **characterised in that** on the fastening device (11) at least one horizontally oriented MRI-compatible receiver flat coils (37, 38) is disposed.

12. Patient support according to claim 11, **characterised in that** one or both vertically oriented, mutually spaced MRI-compatible receiver flat coil(s) (41, 45) are arranged and fastened on the device for fixing a breast (22).

13. Patient support according to claim 1 or 2, **characterised in that** two mutually parallel MRI-compatible receiver flat coils (41, 45), which are vertically oriented and spaced from one another, are disposed in the space (14) that is delimited by topographic projection of the left section (33) or of the right section (32) onto the top side (29) of the base plate (2).

14. Patient support according to one of the preceding claims, **characterised in that** the end (25) of the base plate (2) is connected in an interlocking or integral manner to the end (18) of the support plate (3) by means of an end plate (9) that extends in a horizontal or vertical direction.

15. Patient support according to claim 14, **characterised in that** one or both vertically oriented, mutually spaced MRI-compatible receiver flat coil(s) (41, 45) are arranged and fastened on an inner side (46) of the end plate (9), which is located at the end (18) of the support plate (3).

## Revendications

1. Support de patient possédant une plaque de support (3) supérieure ayant une surface de plaque de support (31) et une plaque de base (2) disposée en dessous de cette dernière et à une certaine distance par rapport à elle, sachant que la plaque de support (3) s'appuie sur la plaque de base (2), sachant qu'un espace intermédiaire (14) est constitué entre la face inférieure de la plaque de support (3) et la face supérieure de la plaque de base (2), sachant que la surface supérieure(31) de la plaque de support (3) présente de son début à son extrémité (18) au moins trois zones de surface consécutives : une zone avant (ZA), suivie d'une zone médiane (ZM) et une zone finale (ZF) suivant cette dernière, qui s'amincit en une mince pièce intermédiaire (30) pour s'élargir ensuite à nouveau en formant ainsi une découpe gauche (33) et une découpe droite (32), qui sont chacune disposée en étant orientée à l'horizontale et de façon symétrique par rapport à l'axe longitudinal et de symétrie (20) de la plaque de support (3), et finalement un dispositif destiné à fixer une poitrine (22), lequel dispositif à fixer une poitrine (22) est disposé dans l'espace intermédiaire (14) de la zone d'une section de surface gauche (39) ou la section de surface droite (40) figurée par la projection cotée de la découpe gauche (33) ou de la découpe droite (32) sur la face supérieure (29) de la plaque de base (2), de façon à s'appuyer à chaque fois directement ou indirectement sur la plaque de base (2), le dispositif destiné à fixer une poitrine (22) étant relié directement ou indirectement en adoptant une forme complémentaire ou un matériau complémentaire avec la plaque de base (2), un appui (23) étant présent **caractérisé par le fait que** le seul appui porteur (23) se trouve dans la zone avant (ZA) dans l'espace intermédiaire (14), lequel appui est, vu dans le sens transversal par rapport au dispositif de l'axe longitudinal, mince par rapport à la largeur de la plaque de support (3), et court vu dans le dispositif de l'axe longitudinal par rapport à la longueur de la plaque de support (3), le dispositif destiné à fixer une poitrine (22) étant disposé de façon à pouvoir être pivoté et arrêté autour d'un axe vertical (42, 43) à l'intérieur de la section de surface gauche (39) et de la section de surface droite (40) figurée de la section gauche (33) et de la section droite (32).

2. Support de patient selon la revendication 1, **caractérisé par le fait qu'**une bobine de surface de réception (48) compatible MRI supérieure gauche (47) et/ou droite agissant sur l'espace (14) délimité sur la face supérieure (29) de la plaque de base (2) par la projection cotée de la découpe gauche (33) ou de la découpe droite (32) est fixée directement ou indirectement sur la plaque de support (3).

3. Support de patient selon la revendication 1 ou 2, **caractérisé par le fait que** le dispositif destiné à fixer une poitrine (22) est relié directement ou indirectement (11) à la plaque de base (2) avec un ou sans dispositif de maintien (41) pour un outil médical au moyen d'un couplage (12, 13) pouvant être au choix adhérent ou de forme complémentaire.

4. Support de patient selon la revendication 1 ou 2, **caractérisé par le fait qu'**un dispositif de fixation (11) destiné à la réception d'au moins une bobine de surface de réception (37, 38) compatible MRI orientée à l'horizontale est disposé dans la section de surface gauche (39) ou la section de surface droite (40) figurée sur la face supérieure (29) de la plaque de base (2) par la projection cotée de la découpe gauche (33) ou de la découpe droite (32), à l'intérieur ou sur ou sous la plaque de base (2).

5. Support de patient selon la revendication 4, **caractérisé par le fait que** le dispositif de fixation (11) peut être déplacé dans le sens horizontal et transversal par rapport à l'axe longitudinal et de symétrie (20).

6. Support de patient selon la revendication 4, **caractérisé par le fait que** le dispositif de fixation (11) présente un certain nombre de dispositifs (12, 16) de réception d'un dispositif destiné à fixer une poitrine (22) avec ou sans dispositif de maintien (41) pour un outil médical.

7. Support de patient selon la revendication 4, **caractérisé par le fait que** le dispositif destiné à fixer une poitrine (22) avec ou sans dispositif de maintien (41) pour un outil médical est relié au dispositif de fixation (11) au choix au moyen d'un couplage (12, 13) pouvant être ouvert ou fermé, adhérent ou de forme complémentaire.

8. Support de patient selon les revendications 1, 3 à 5 ou 2 à 5, **caractérisé par le fait que** dans l'espace intermédiaire d'une section de surface gauche (39) et de la section de surface droite (32) figuré sur la face supérieure (29) de la plaque de base (2) par la projection cotée de la découpe gauche (33) ou de la découpe droite (32) est disposé un dispositif de maintien (41) pour un outil médical, ce dispositif de maintien (41) pour l'outil médical s'appuyant directement ou indirectement sur la plaque de base (2).

9. Support de patient selon la revendication 8, **caractérisé par le fait que** le dispositif de maintien (41) pour un outil médical est relié par forme complémentaire ou par matériau complémentaire au dispositif destiné à fixer une poitrine (22).

10. Support de patient selon les revendications 1, 3 à 9 ou 2 à 9, **caractérisé par le fait qu'**au moins une bobine de surface de réception (37, 38) compatible MRI gauche et/ou droite orientée à l'horizontale est disposée dans la section de surface gauche (39) ou la section de surface droite (40) figurée par la projection cotée de la découpe gauche (33) ou de la découpe droite (32) sur la face supérieure (29) de la plaque de base (2), à l'intérieur ou sur ou sous la plaque de base (2).

11. Support de patient selon la revendication 4, **caractérisé par le fait que** sur le dispositif de fixation (11) est disposée au moins une bobine de surface de réception (37, 38) compatible MRI orientée à l'horizontale.

12. Support de patient selon la revendication 11, **caractérisé par le fait qu'**une ou des bobines de surface de fixation (41, 45) compatible MRI, l'une d'entre elle ou les deux étant orientées à la verticale à une certaine distance l'une de l'autre, sont disposées et fixées sur le dispositif destiné à fixer une poitrine (22).

13. Support de patient selon la revendication 1 ou 2, **caractérisé par le fait que** dans l'espace (14) délimité par la projection cotée de la découpe gauche (33) ou de la découpe droite (32) sur la face supérieure (29) de la plaque de base (2) sont disposées deux bobines de surface (41, 45) compatibles MRI disposées à la verticale parallèlement l'une à l'autre et à une certaine distance l'une de l'autre.

14. Support de patient selon une des revendications précédentes, **caractérisé par le fait que** l'extrémité (25) de la plaque de base (2) est reliée par forme complémentaire ou par matériau complémentaire à l'extrémité (18) de la plaque de support (3) au moyen d'une plaque finale (9) s'étendant dans le sens horizontal et dans le sens vertical.

15. Support de patient selon la revendication 14, **caractérisé par le fait qu'**une ou deux bobines de surface (41, 45) compatibles MRI disposées l'une ou l'autre ou les deux à la verticale à une certaine distance l'une de l'autre sont fixées sur une face interne (46) de la plaque finale (9) se trouvant à l'extrémité (18) de la plaque de support (3).
